(19) Europäisches Patentamt
European Patent Office
Office européen des brevets

(11) **EP 3 165 157 A1**

(12) **EUROPEAN PATENT APPLICATION**
published in accordance with Art. 153(4) EPC

(43) Date of publication:
**10.05.2017 Bulletin 2017/19**

(51) Int Cl.:
*A61B 5/022* [(2006.01)]  *A61B 5/0245* [(2006.01)]
*A61B 5/026* [(2006.01)]  *A61B 5/08* [(2006.01)]
*A61B 5/11* [(2006.01)]

(21) Application number: **15814026.9**

(22) Date of filing: **30.06.2015**

(86) International application number:
**PCT/JP2015/068787**

(87) International publication number:
**WO 2016/002759 (07.01.2016 Gazette 2016/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **01.07.2014 JP 2014136082
12.03.2015 JP 2015049591**

(71) Applicants:
• **Cyberdyne Inc.**
**Tsukuba-shi, Ibaraki 305-0818 (JP)**

• **University of Tsukuba**
**Tsukuba-shi, Ibaraki 305-8577 (JP)**

(72) Inventor: **SANKAI, Yoshiyuki**
**Tsukuba-shi**
**Ibaraki 305-8577 (JP)**

(74) Representative: **Hoffmann Eitle**
**Patent- und Rechtsanwälte PartmbB**
**Arabellastraße 30**
**81925 München (DE)**

(54) **BIOLOGICAL INFORMATION DETECTION DEVICE, SEAT WITH BACKREST, AND CARDIOPULMONARY FUNCTION MONITORING DEVICE**

(57)    Provided are a biological-information detecting apparatus and a seat with a backrest capable of detecting biological information about a measurement subject with high accuracy while reducing burdens on the measurement subject in non-contact and non-retraining manner. According to the embodiment, the biological-information detecting apparatus includes first blood-flow measuring means 2 provided at a seat surface of a seat, the first blood-flow measuring means measuring a condition of a blood flow of a popliteal artery of a measurement subject seated in the seat, second blood-flow measuring means 3 provided at a back of the seat, the second blood-flow measuring means measuring a condition of a blood flow of a thoracic aorta of the measurement subject, and blood-pressure calculation means determining a pulse-wave propagation velocity and a degree of arteriosclerosis from blood flow data acquired by the first blood-flow measuring means 2 and blood flow data acquired by the second blood-flow measuring means 3 and calculating blood pressures of the popliteal artery and the thoracic aorta of the measurement subject based on the pulse-wave propagation velocity and the degree of arteriosclerosis.

FIG.1

**Description**

Technical Field

The present invention relates to a biological-information detecting apparatus for detecting biological information about a measurement subject and to a seat with a backrest. The present invention also relates to a cardiopulmonary-function monitoring apparatus for monitoring cardiopulmonary functions, such as breathing and heartbeat.

Background Art

In general, worsening of the health condition of a vehicle driver can exert a bad influence on driving of the vehicle, and therefore it is desirable to detect the worsening of the health condition in advance and to take some measures against it. For that purpose, a health care system has been proposed which allows the health condition of the driver to be continuously monitored during the driving.

For example, biological information on the driver is detected by letting the driver grasp an electrode provided on the steering wheel in the vehicle (see PTL 1) or by winding or attaching a cuff around an arm or a fingertip of the driver (see PTL 2).

However it is troublesome for the driver to wind the cuff, and also the driver undergoes pressure from the cuff. Moreover, letting the driver grasp the electrode provided on the steering wheel restricts the orientation of the driver.

Thus, the related art has a problem in that a heavy burden is imposed on vehicle drivers in detecting biological information on the vehicle drivers.

To reduce the problem of the burden on the vehicle driver, methods of diagnosis by measuring the heartbeat and breathing by emitting a microwave in non-contact manner have been proposed (for example, see PTLs 3 and 4). However, even the non-contact methods for detecting biological information as disclosed in PTLs 3 and 4 need vehicle drivers to wear a sensor or the like, thus having the problem of heavy burden.

Since drivers of taxies, buses, and other vehicles absolutely need health care and relatively frequently get on and off because of their operations, it is desirable to constantly monitor the blood-pressure values without restraining their hands, feet, or the like.

In medical facilities etc., to detect cardiopulmonary functions including the heart rate and breathing rate of hospital patients, sensors of medical devices, such as a respirator and an electrocardiograph, are brought into direct-contact with the patients, and the sensors and a main apparatus are connected with cables. However, the method of direct contact of the sensors and the patients will restrain the motion of the patients and cause a feeling of pressure. Moreover, if the sensors have come off because of roll-over during sleeping or another cause, the cardiopulmonary functions cannot be detected.

PTL 1: Japanese Unexamined Patent Application Publication No. 2007-290504
PTL 2: International Publication No. WO 2007/46283
PTL 3: Japanese Unexamined Patent Application Publication No. 2008-99849
PTL 4: Japanese Unexamined Patent Application Publication No. 2014-105
PTL 5: Japanese Unexamined Patent Application Publication No. 2004-174168

Summary of Invention

The present invention has been made in view of the above situations of the related art, and it is an object of the present invention to provide a biological-information detecting apparatus and a seat with a backrest capable of detecting biological information about a measurement subject with high accuracy while reducing burdens on the measurement subject in non-contact and non-retraining manner. Another object of the present invention is to provide a cardiopulmonary-function monitoring apparatus capable of monitoring cardiopulmonary functions without contact with a human body.

According to one aspect of the present invention, there is provided a biological-information detecting apparatus including first blood-flow measuring means provided at a seat surface of a seat, the first blood-flow measuring means measuring a condition of a blood flow of a popliteal artery of a measurement subject seated in the seat, second blood-flow measuring means provided at a back of the seat, the second blood-flow measuring means measuring a condition of a blood flow of a thoracic aorta of the measurement subject, and blood-pressure calculation means determining a pulse-wave propagation velocity and a degree of arteriosclerosis from blood flow data acquired by the first blood-flow measuring means and blood flow data acquired by the second blood-flow measuring means and calculating blood pressures of the popliteal artery and the thoracic aorta of the measurement subject based on the pulse-wave propagation velocity and the degree of arteriosclerosis.

According to one aspect of the present invention, the biological-information detecting apparatus further includes a database storing the blood flow data acquired from the first blood-flow measuring means and the second blood-flow measuring means. The blood-pressure calculation means calculates the blood pressures of the popliteal artery and the thoracic aorta in chronological order based on the individual blood flow data stored in the database over a predetermined period of time to monitor a health condition of the measurement subject.

According to one aspect of the present invention, the biological-information detecting apparatus further includes cardiopulmonary-function measuring means provided at the back of the seat, the cardiopulmonary-function measuring means measuring a heart rate and a breathing rate of the measurement subject. The blood-pressure calculation means individually corrects the blood pressures of the popliteal artery and the thoracic aorta based on the heart rate and the breathing rate so that time differences in the waveform of the pressure of the popliteal artery and in the waveform of the thoracic aorta of the measurement subject become substantially constant.

According to one aspect of the present invention, the database stores heart rates and breathing rates acquired from the cardiopulmonary-function measuring means, and the blood-pressure calculation means corrects the blood pressures of the popliteal artery and the thoracic aorta based on the heart rates and the breathing rates stored in the database over the predetermined period.

According to one aspect of the present invention, a seat with a backrest includes the biological-information detecting apparatus.

According to one aspect of the present invention, there is provided a cardiopulmonary-function monitoring apparatus including an oscillator producing a microwave, a transmitting antenna transmitting the microwave produced by the oscillator, a receiving antenna receiving a reflected wave from a monitored object, a mixer mixing the microwave produced by the oscillator with the reflected wave to output a signal based on a difference between the microwave and the reflected wave, an analog-to-digital converter converting the signal output from the mixer from analog to digital, and a calculation control unit performing a Fourier transform on the signal output from the analog-to-digital converter and performing a filtering process on the Fourier transformed signal to calculate a heart rate and a breathing rate.

According to one aspect of the present invention, the calculation control unit calculates the heart rate based on a frequency whose amplitude is maximum in a frequency distribution after a filtering process using a first filter corresponding to a heartbeat and calculates the breathing rate based on a frequency whose amplitude is maximum in a frequency distribution after a filtering process using a second filter corresponding to breathing.

According to one aspect of the present invention, the cardiopulmonary-function monitoring apparatus further includes an informing unit giving an alarm of occurrence of abnormality. When a difference between the calculated heart rate or breathing rate and a reference value is greater than or equal to a predetermined value, the calculation control unit instructs the informing unit to given an alarm of the occurrence of abnormality.

According to one aspect of the present invention, the cardiopulmonary-function monitoring apparatus further includes a storage unit storing heart rates and breathing rates calculated by the calculation control unit. The calculation control unit uses an average value of the heart rates or the breathing rates stored in the storage unit as the reference value.

According to one aspect of the present invention, the microwave produced by the oscillator has a frequency of 10 MHz.

According to one aspect of the present invention, the transmitting antenna and the receiving antenna are mounted on a ceiling or a side wall surface of a space in which the monitored object stays in a recumbent position, a seated position, or a standing position.

According to one aspect of the present invention, the transmitting antenna and the receiving antenna are configured to be attachable to or detachable from a ceiling or a wall surface of a space in which the monitored object stays in a recumbent position, a seated position, or a standing position.

According to one aspect of the present invention, at least the transmitting antenna and the receiving antenna are built in another installation instrument.

According to one aspect of the present invention, at least the transmitting antenna and the receiving antenna are built in a portable device.

Advantageous Effects of Invention

The present invention allows biological information about a measurement subject to be detected with high accuracy while reducing burdens on the measurement subject in non-contact and non-restraining manner.

Brief Description of Drawings

[Fig. 1] Fig. 1 is a block configuration diagram of a biological-information detecting apparatus according to a first embodiment of the present invention.

[Fig. 2] Fig. 2 is an internal configuration diagram of a first blood-flow measurement sensor and a second blood-flow measurement sensor.

[Fig. 3] Fig. 3 is a vertical cross-sectional diagram of a sensing-unit mount structure in an enlarged view.

[Fig. 4] Fig. 4 is a diagram for illustrating the principle of a method for measuring blood flow.

[Fig. 5] Fig. 5 is a graph showing the relationship between the state of light absorption and different wavelengths of a laser beam and different degrees of oxygen saturation of blood.

[Fig. 6] Figs. 6A to 6C are waveform charts showing the waveforms of signals detected by individual photosensor units.

[Fig. 7] Fig. 7 is a flowchart for illustrating a PWV control process based on detection signals from the individual photosensor units.

[Fig. 8] Fig. 8 is a block diagram illustrating the internal configuration of a cardiopulmonary function sensor.

[Fig. 9] Fig. 9 is a schematic configuration diagram of a cardiopulmonary-function monitoring apparatus according to a second embodiment.

[Fig. 10] Figs. 10A and 10B are graphs illustrating the behaviors of the heart and the lungs.

[Fig. 11] Fig. 11 is a diagram illustrating an installation example of transmitting and receiving antennas of a cardiopulmonary-function monitoring apparatus.

Description of Embodiments

Embodiments of the present invention will be described hereinbelow with reference to the drawings.

[Configuration of Biological-Information Detecting Apparatus]

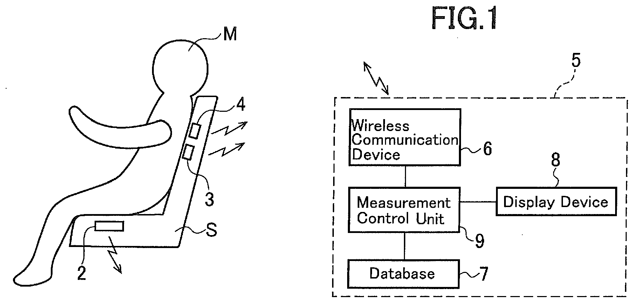

Fig. 1 is a block configuration diagram of a biological-information detecting apparatus 1 according to a first embodiment of the present invention. As illustrated in Fig. 1, the biological-information detecting apparatus 1 includes a first blood-flow measurement sensor 2, a second blood-flow measurement sensor 3, a cardiopulmonary function sensor 4, and an external unit 5 mounted to a vehicle seat S and is configured to wirelessly transmit detection results from the sensors 2 to 4 to the external unit 5.

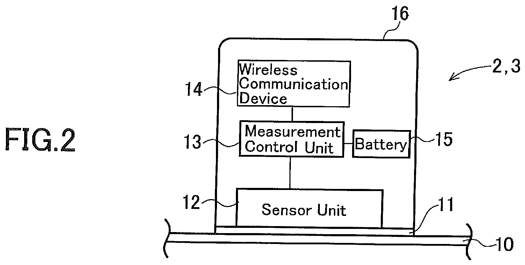

The first blood-flow measurement sensor 2 and the second blood-flow measurement sensor 3 individually measure blood flows at positions facing the surface of the skin in target regions of the measurement subject. The first blood-flow measurement sensor 2 and the second blood-flow measurement sensor 3 have the same configuration, in each of which a sensor unit 12 (Fig. 2) including an optical sensor that measures a blood flow in a blood vessel without contact is built.

In this embodiment, the first blood-flow measurement sensor 2 is disposed at the seating surface of the vehicle seat S, emits near infrared light toward the hip or thigh of a measurement subject M seated in the vehicle seat S, and receives reflected light to detect displacement (vascular properties) of the popliteal artery and tissue around the popliteal artery due to the blood flow. The second blood-flow measurement sensor 3 is disposed at the back of vehicle seat S, emits near infrared light toward the back of the measurement subject M seated in the vehicle seat S, and receives reflected light to detect the vascular properties of the thoracic aorta.

The cardiopulmonary function sensor 4 is disposed at the back of the vehicle seat S, emits a microwave toward the back of the measurement subject M seated in the vehicle seat S, and receives reflected light to detect the heart rate and the breathing rate.

The external unit 5 includes a wireless communication device 6 that receives measurement data, which is the results of sensing by the first blood-flow measurement sensor 2, the second blood-flow measurement sensor 3, and the cardiopulmonary function sensor 4, a database 7 that stores the measurement data, a display device 8 that displays various processing results based on the individual measurement data, and a control unit 9 responsible for control of all the above devices.

[Internal Configuration of First Blood-Flow Measurement Sensor 2 and Second Blood-Flow Measurement Sensor 3]

The configuration of the first blood-flow measurement sensor 2 (the second blood-flow measurement sensor 3) in Fig. 2 will be described hereinbelow. The first blood-flow measurement sensor 2 (the second blood-flow measurement sensor 3) has a flexible substrate 11 on one surface of a thin resin sheet-like board 10. A sensor unit 12, a measurement control unit 13, a wireless communication device 14, and a chargeable buttery 15 are mounted on the flexible substrate 11, all of which are covered with a casing 16 made of resin or the like for protection.

In the first blood-flow measurement sensor 2 (the second blood-flow measurement sensor 3), the other surface of the sheet-like board 10 is a measurement surface opposed to the measurement target region (close thereto without contact). By appropriately opposing the measurement surface on the other surface of the sheet-like board to the surface of the skin in the measurement target region, the displacement of a blood vessel and tissue around the blood vessel due to the blood flow in the measurement target region (vascular properties) can be measured without contact using the sensor unit 12.

In the first blood-flow measurement sensor 2 (the second blood-flow measurement sensor 3), the measurement control unit 13 makes a current from the battery 15 pass through a light-emitting section of the sensor unit 12 to cause light emission and reads a light-receiving signal from a light-receiving section that has received light propagating over the surface of the skin. The wireless communication device 14 communicates with the control unit 9 of the external unit 5 (Fig. 1) and wirelessly transmits the light-receiving signal from the light-receiving section to the control unit.

Since the first blood-flow measurement sensor 2 (the second blood-flow measurement sensor 3) is a wireless unit capable of wireless communication with the external unit 5, it can freely be moved to the measurement target region. The chargeable battery 15 of the first blood-flow measurement sensor 2 (the second blood-flow measurement sensor 3) is appropriately charged with electricity in non-use during which no blood flow measurement is performed.

[Internal Configuration of Sensor Unit 12]

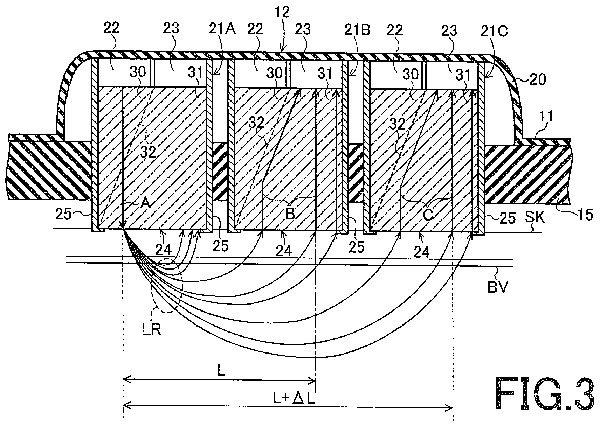

The internal configuration of the sensor units of the first blood-flow measurement sensor 2 and the second blood-flow measurement sensor 3 will be described hereinbelow. Fig. 3 is a vertical cross-sectional diagram of the mount structure of the sensor units 12 in enlarged view.

As illustrated in Fig. 3, each of the sensor units 12 includes three photosensor units 21A to 21C disposed in line inside a dome-shaped flexible wiring board 20.

When the measurement subject is seated in the vehicle seat S, the individual photosensor units 21A, 21B, and 21C are held such that a light-emitting surface and a light-receiving surface at each distal end come close to (or into contact with) the skin surface of the measurement subject. The photosensor units 21A, 21B, and 21C have the same configuration, and like components are given like signs.

The photosensor unit 21A includes a light-emitting section 22, a light-receiving section 23, and an optical-path separating member 24. The light-emitting section 22 is constituted of a laser diode that emits a laser beam (an outgoing beam) A onto a skin surface. The light-receiving section 23 is constituted of a light-receiving element that outputs an electrical signal according to the amount of transmitted light received. The optical-path separating member 24 is constituted of, for example, a holographic optical element (HOE) using a hologram and is configured such that a refractive index for a laser beam A emitted from the light-emitting section 22 toward the measurement target region and the refractive indices of incoming beams B and C coming through the measurement target region toward the light-receiving section differ.

A housing 25 formed in a cylindrical shape is fit on the outer periphery of each optical-path separating member 24. The upper surfaces of the light-emitting section 22 and the light-receiving section 23 are mounted on the lower surface of the flexible wiring board 20. The flexible wiring board 20 has a wiring pattern connected to the measurement control unit 13 (Fig. 2). The wiring pattern is electrically connected, at positions corresponding to the photosensor units 21A to 21C, to connecting terminals of the light-emitting section 22 and the light-receiving section 23 by soldering or the like. The flexible wiring board 20 is configured to deflect according the surface shapes of the distal ends of the photosensor units 21A to 21C when the distal ends come into contact with the measurement target region.

For blood flow measurement, the measurement control unit 13 makes the light-emitting section 22 of the photosensor unit 21A emit the laser beam A. At that time, the laser beam emitted from the light-emitting section 22 is output at a wavelength $\lambda$ ($\lambda \approx 805$ nm), which is not influenced by the degree of oxygen saturation.

Each of the photosensor units 21A to 21C is held in a state in which the distal end (the end face of the optical-path separating member 24) is in contact with the measurement target region of the measurement subject. The laser beam A emitted from the light-emitting section 22 passes through the optical-path separating member 24 into a skin surface SK in the vertical direction. Inside the skin surface SK (in the body), the laser beam A travels toward the center and also propagates to the periphery along the skin surface SK, with the incident position as the starting point. The light propagation paths LR of the laser beam A are arc-shaped in side view and return to the skin surface SK through a blood vessel BV.

The light that has passed through the light propagation paths LR reaches the receiving-side photosensor units 21B and 21C while changing in the amount of light transmitted according to the amount or density of red blood cells contained in blood flowing through the blood vessel BV. Since the laser beam A gradually decreases in the amount of light transmitted during propagation through the human body, the light reception level of the light-receiving section 23 decreases in proportion to the distance as the incident position of the laser beam A is separated from the starting point. Accordingly, the amount of received transmitted light changes also with the distance from the incident position of the laser beam A.

In Fig. 3, assuming that the photosensor unit 21A at the left end is a light-emitting point, the photosensor unit 21A itself, the photosensor unit 21B on its immediate right, and the photosensor unit 21C on the right next to it are light-receiving points (measurement points).

The optical-path separating member 24 is formed to make the laser beam A travel in a straight line and to lead the incoming beams B and C to the light-receiving section by, for example, changing the density distribution of a transparent acrylic resin. The optical-path separating member 24 includes an exiting-side transmission region 30 that transmits the laser beam A emitted from the light-emitting section 22 from the proximal end (the upper surface in Fig. 3) to the distal end (the lower surface in Fig. 3), an incident-side transmission region 31 that transmits light propagated in the human body from the distal end (the lower surface in Fig. 3) to the proximal end (the upper surface in Fig. 3), and a refraction region 32 formed between the exiting-side transmission region 30 and the incident-side transmission region 31.

The refraction region 32 has the property of transmitting the laser beam A but reflecting light that has passed through a blood flow (the incoming beams B and C). The refraction region 32 is formed by, for example, changing the density of an acrylic resin, providing a metal thin film in this region, or dispersing metal fine particles. This allows all light incident from the distal end of the optical-path separating member 24 to be collected to the light-receiving section 23.

[Principle of Method for Measuring Blood Flow]

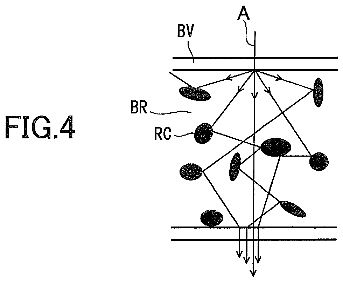

Fig. 4 is a diagram for illustrating the principle of a method for measuring a blood flow. As shown in Fig. 4, when the laser beam A is emitted from the outside to blood in the blood vessel BV, the laser beam A that has entered a blood layer BR travels in the blood as light containing a reflected-and-scattered-light component due to normal red blood cells RC and a reflected-and-scattered-light component due to attached blood clots.

Since an influence exerted upon the light in the course of transmission in the blood layer changes from moment to moment according to the state of the blood, changes in the various properties of the blood can be observed by continuously measuring the amount of transmitted light (or the amount of reflected light) and observing the change in the amount of light.

Since the amount of oxygen consumed in the body increases as the momentum of the measurement subject increases, the state of the blood flow caused by the hematocrit of red blood cells that convey oxygen and the degree of oxygen saturation of the blood appears as a change in the amount of light.

Here, changes in hematocrit (Hct: the volume percentage of red blood cells per unit volume, that is, the volume concentration of red blood cells per unit volume, also expressed as Ht) and so on are also factors related to a change in the density of hemoglobin, exerting an influence on a change in light amount. The fundamental principle of this embodiment is to measure the state of a blood flow using changes in optical path and the amount of transmitted light due to the blood flow using the laser beam A.

Furthermore, the fundamental configuration thereof will be described. The optical characteristics of blood depend on blood cell components (in particular, hemoglobin in red blood cells). Since red blood cells have a property in which hemoglobin easily combines with oxygen, it also serves as a conveyor of oxygen. The degree of oxygen saturation of blood is a numerical value indicating what percentage of hemoglobin in the blood combines with oxygen. The degree of oxygen saturation has correlation with the partial pressure of oxygen ($PaO_2$) in arterial blood and is an important indicator of a breathing function (gas exchange).

It is known that a high partial pressure of oxygen increases the degree of oxygen saturation. A change in the degree of oxygen saturation also changes the amount of light transmitted in the blood. For that reason, in measuring a blood flow, higher accuracy measurement can be achieved by eliminating the influence of the degree of oxygen saturation.

One factor that influences the partial pressure of oxygen ($PaO_2$) is an alveolar ventilation volume. Other factors include environments, such as atmospheric pressure and a fraction of inspiratory oxygen ($FiO_2$), a ventilation/blood-flow ratio, a gas diffusing capacity, a shunt index, and gas exchange in alveoli.

The control unit 9 of the external unit 5 detects the state of the blood flow by executing a PWV control process, to be described later, based on measurement data according to the amount of transmitted light (light intensity) generated by the light-receiving sections 23 of the photosensor units 21A to 21C.

The laser beam A from the light-emitting section 22 is intermittently emitted at predetermined time intervals (for example, 10 Hz to 1 MHz) as pulsed light or is emitted as continuous light. In that case, with pulsed light, a blinking frequency, which is the frequency of blinking of the pulsed light, is determined according to a blood flow rate, and the measurement is performed continuously or at a measurement sampling frequency twice or more the blinking frequency. With continuously light, the measurement is performed using a measurement sampling frequency determined according to the blood flow rate.

Hemoglobin (Hb) in blood chemically reacts with oxygen in the lungs by breathing to produce $HbO_2$, taking oxygen into the blood. However, the degree of oxygen taken into the blood (the degree of oxygen saturation) differs subtly according to the state of breathing and so on. In other words, in this embodiment, a phenomenon in which the degree of oxygen saturation changes the optical absorptance of blood irradiated with light is found. Since this phenomenon is a disturbing element in the measurement of a blood flow using the laser beam A, the influence due to the degree of oxygen saturation needs to be eliminated.

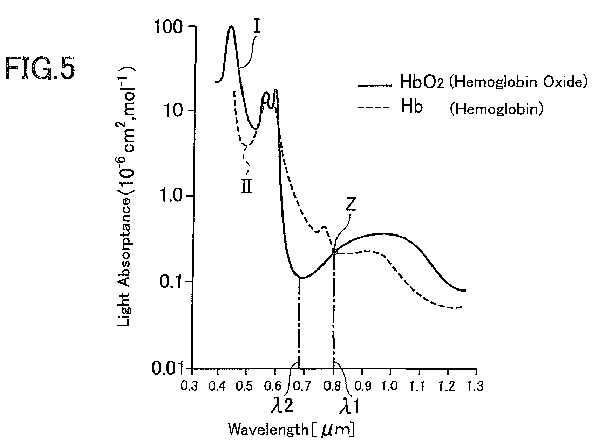

Fig. 5 is a graph showing the relationship between the state of light absorption and different wavelengths of a laser beam and different degrees of oxygen saturation of blood. In the body, hemoglobin contained in red blood cells is classified into hemoglobin oxide combined with oxygen ($HbO_2$: graph I [indicated by the solid line]) and hemoglobin that is not oxidized (Hb: graph II [indicated by the broken line]). The optical absorptance differs significantly between the two states. For example, blood containing a large amount of oxygen is fresh blood in vivid color. In contrast, venous blood is dull and dark because oxygen is released. The states of light absorption change in a wide light wavelength region, as shown in graphs I and II in Fig. 5.

By selecting a specific wavelength from graphs I and II in Fig. 5, blood flow measurement can be performed by applying light to the blood without an influence on the optical absorptance even if the degree of oxygen saturation of hemoglobin in the red blood cells changes significantly due to oxygen metabolism or the like in the body.

The optical absorptance is low in some wavelength region regardless of the degree of oxygen saturation of hemoglobin in the red blood cells. For that reason, whether the light is likely to pass through a blood layer is determined by the wavelength $\lambda$. Thus, using light with a predetermined wavelength range (for example, $\lambda$ = about 800 nm to about 1,300 nm) allows blood flow measurement with reduced influence of the degree of oxygen saturation.

Thus, using a laser beam A with a wavelength region from substantially 600 nm to 1,500 nm allows the optical absorptance of hemoglobin (Hb) to be sufficiently low for practical use. Furthermore, since this region contains an isosbestic point Z, measurement points of two or more wavelengths can be used, which can be regarded as an isosbestic point in terms of calculation. In other words, this allows specifications in which the degree of oxygen saturation has no influence.

In other wavelength regions, for example, in a wavelength region of $\lambda$ = less than 600 nm, the optical absorptance increases to decrease the S/N ratio, and in a wavelength region higher than $\lambda$ = 1,500 nm, the light-receiving sensitivity of the light-receiving section is insufficient, hindering high-accuracy measurement because of disturbance, such as the other constituents of the blood.

For this reason, this embodiment uses a light-emitting device constituted of a wavelength-tunable semiconductor laser as the light-emitting section 22, in which the wavelength of the laser beam A emitted from the light-emitting section 22 is set to two kinds, that is, $\lambda 1$ = 805 nm (a first beam) corresponding to the isosbestic point Z in graphs I and II and a wavelength $\lambda 2$ = 680 nm (a second beam) having the lowest optical absorptance in graph I.

Here, a method for detecting concentrations R, Rp, and Rpw of red blood cells based on the amounts of transmitted laser beam A propagating through the light propagation paths LR (Fig. 3) will be described.

An operational expression (1) for the concentration R of red blood cells using a conventional one-point one-wavelength method is expressed as the following equation.

$$R = \log 10(I_{in}/I_{out}) = f(I_{in}, L, Ht) \quad \cdots (1)$$

In the method using Exp. (1), the concentration of red blood cells is expressed as a function of the incident transmitted light quantity $I_{in}$ of the laser beam A emitted from the light-emitting section 22, the distance (optical path length) L between the light-emitting section 22 and the light-receiving section 23, and the hematocrit (Ht) described above. For that reason, in determining the concentration of red blood cells using the method using Exp. (1), the concentration of red blood cells is varied by the three factors, which makes it difficult to accurately measure the concentration of red blood cells.

An operational expression (2) for the concentration Rp of red blood cells using a two-point one-wavelength method according to this embodiment is expressed as the following equation.

$$Rp = \log 10\{I_{OUT}/(I_{out} - \Delta I_{OUT})\} = \Phi(\Delta L, Ht) \quad \cdots (2)$$

In the method using Exp. (2), since the laser beam A is received at two points (the light-receiving sections of the photosensor units 21B and 21C) at different distances from the laser beam A, as illustrated in Fig. 3, the concentration of red blood cells is expressed as a function of the distance $\Delta L$ between the two light-receiving sections 23 and the hematocrit (Ht), described above. For that reason, in determining the concentration of red blood cells using the method using Exp. (2), the concentration of red blood cells is measured as a value in which the hematocrit (Ht) is a factor because, among the two factors, the distance $\Delta L$ between the light-receiving sections 23 is known in advance. Thus, this calculation method allows the concentration of red blood cells to be accurately measured as a measured value based on hematocrit (Ht).

Furthermore, an operational expression (3) for the concentration Rpw of red blood cells using a two-point two wavelength method according to a modification of this embodiment is expressed as the following equation.

$$Rpw=[\log10\{I_{OUT}/(I_{out}-\Delta I_{OUT})\}\lambda1]/[\{\log10\{I_{OUT}/(I_{OUT}-\Delta I_{OUT})\}\lambda2]=\xi(Ht) \cdots(3)$$

In the method using Exp. (3), the concentration of red blood cells is determined as a function containing only hematocrit (Ht) by setting the wavelength of the laser beam A emitted from the light-emitting section to different wavelengths $\lambda1$ and $\lambda2$ (in this embodiment, $\lambda1$ = 805 nm, $\lambda2$ = 680 nm). For that reason, this calculation method allows the concentration of red blood cells to be accurately measured as a measured value based on hematocrit (Ht).

For propagation of light, the light propagation path LR increase, and therefore the light transmittance decreases, with an increasing distance in the radial direction from the starting point from which the laser beam A is emitted. For that reason, the detected light-receiving level (transmitted light quantity) of the photosensor unit 21B next to the light-emitting-side photosensor unit 21A a predetermined distance away therefrom is low, and the detected light-receiving level (transmitted light quantity) of the photosensor unit 21C next thereto is lower than the light-receiving level of the photosensor unit 21B. The light-receiving section of the light-emitting-side photosensor unit 21A also receives light from the skin surface. The control unit 9 of the external unit 5 causes detection signals according to the intensities of light received by the plurality of photosensor units 21A to 21C to be stored in the database 7 in chronological order.

Furthermore, letting $I_{out}$ in Exp. (2) or (3) be detection signals (signals according to the amounts of transmitted light received) output from the individual photosensor units 21A to 21C, described above, allows the concentration of red blood cells to be accurately determined as a measured value (a value that is not influenced by the degree of oxygen saturation) according to hematocrit (Ht).

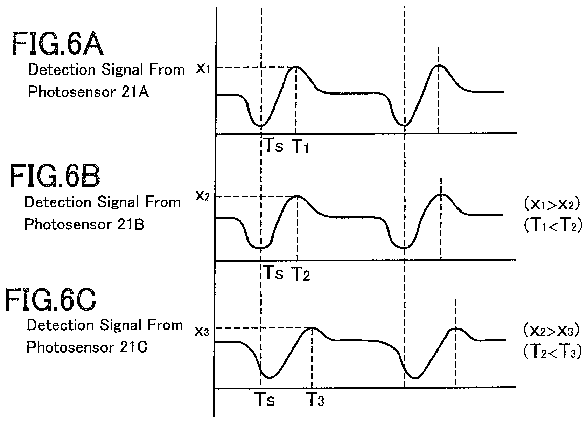

Figs. 6A to 6C are waveform charts illustrating the waveforms of detection signals from the individual photosensor units 21A to 21C. As shown in Figs. 6A to 6C, by comparing the waveforms of the detection signals from the light-receiving sections starting from time Ts at which the laser beam A is emitted from the light-emitting section 22, the phase differences T1 to T3 between the values at the emission time Ts and the highest values of the detection signals from the light-receiving sections are obtained.

The phase differences T1 to T3 have the relation of T1 < T2 < T3 and change according to the pulse-wave propagation velocity. Approximations $\Delta t1$ and $\Delta t2$ of PWV are obtained from the phase differences, T2 - T1 = $\Delta t1$ and T3 - T1 = $\Delta t2$, between the detection signals from the individual photosensor units 21A to 21C.

[PWV Control Process by Control Unit]

A pulse wave velocity (PWV) control process executed by the control unit 9 of the external unit 5 will be described hereinbelow.

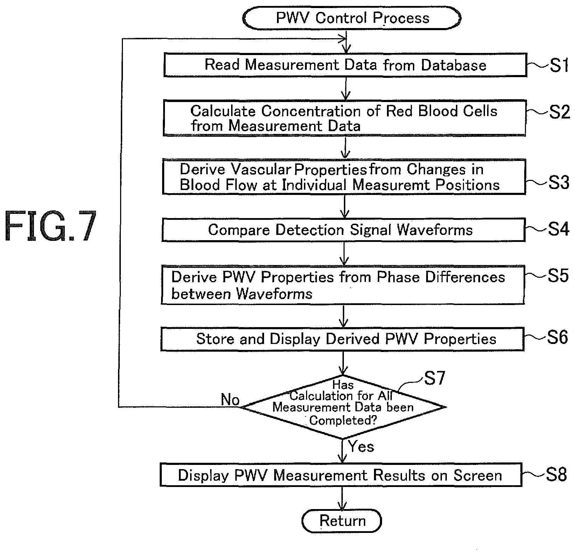

Fig. 7 is a flowchart for illustrating a PWV control process based on the detection signals from the individual photosensor units 21A to 21C. In Fig. 7, at S1, the control unit 9 reads measurement data (measurement data based on the amounts of the transmitted light according to the blood flow) stored in the database 7. Next at S2, the control unit 9 calculates concentration of red blood cells, Rp or Rpw, using the measurement data and operational expression (2) or (3), described above.

Next at S3, the control unit 9 obtains changes in the displacement of the blood vessel and tissue around the blood vessel from the change in the concentration of red blood cells at individual measurement positions due to the blood flow and derives blood flow rates at the individual measurement positions on the basis of the displacement of the blood vessel and the tissue around the blood vessel.

Subsequently, at S4, the control unit 9 compares detection signal waveforms (or waveforms of inner-wall displacement data corresponding to changes in blood flow) output from the individual photosensor units 21A to 21C.

At S5, the control unit 9 calculates the values of PWV, $\Delta t1$ and $\Delta t2$, from the phase differences between the waveforms of the detection signals, T2 - T1 = $\Delta t1$ and T3 - T1 = $\Delta t2$, as shown in Figs. 6A to 6C. Furthermore, the control unit 9 calculates a pulse-wave propagation velocity based on the values of PWV, and derives vascular properties of the measurement target region corresponding to the pulse-wave propagation velocity (the percentage of the elasticity of the blood vessel, the amount of plaque in the blood vessel, the percentage of arteriosclerosis) from the database 7 to derive the degree of arteriosclerosis of the blood vessel in the measurement target region.

Subsequently, at S6, the control unit 9 stores the pulse-wave propagation velocity based on the values of PWV and the degree of arteriosclerosis of the blood vessel in the database and displays the pulse-wave propagation velocity based on the values of PWV and the degree of arteriosclerosis of the blood vessel on the display device 8. In addition, the control unit 9 obtains the phase difference between measurement data from the first blood-flow measurement sensor and the second blood-flow measurement sensor and stores it as a calculation result.

Next at S7, the control unit 9 determines whether calculation of PWVs and detection of the pulse-wave propagation velocities for all of measurement data from the photosensor units 21A to 21C (for example, all of one week's data) have been completed. If at S7 the calculation of PWVs and detection of the pulse-wave propagation velocities for all of measurement data have not been completed, the control unit 9 returns to S21 and repeats the processes from S21 onward.

If at S7 the calculation of PWVs and detection of the pulse-wave propagation velocities for all measurement data have completed, the control unit 9 goes to S8, at which the results of measurement of PWVs for all measurement data and the pulse-wave propagation velocities input from the control unit 9 of the external unit 5 are displayed on the display screen of the display device 8. This allows checking of the PWV measurement results and the pulse-wave propagation velocities based on all measurement data regarding the measurement subject, acquired over a predetermined period (for example, one week) of life.

[Method for Calculating Blood Pressure]

As described above, the control unit 9 of the external unit 5 can determine a pulse-wave propagation velocity and the degree of arteriosclerosis on the basis of the vascular properties (the displacement of the blood vessel and tissue around the blood vessel due to the blood flow) of the popliteal artery and the thoracic aorta of the measurement subject.

The control unit 9 can determine the blood pressure (arterial blood pressure) of the measurement subject by calculation based on the pulse-wave propagation velocity and the degree of arteriosclerosis.

In other words, it is known that the following relational expression holds by using the degree of arteriosclerosis, where c is the pulse-wave propagation velocity, and P is the arterial blood pressure. This indicates that the pulse-wave propagation velocity depends on the hardness of the blood vessel and also the blood pressure. In the following mathematical expression, $\rho$ is a constant indicating the density of blood, and $\beta$ is a constant (a stiffness parameter) indicating the hardness of the blood vessel.

[Expression 1]

$$c^2 = \frac{\beta P}{2\rho}$$

Thus, the control unit 9 can calculate the blood pressures of the popliteal artery and the blood pressure of the thoracic aorta (arterial blood pressure P) of the measurement subject.

[Configuration of Cardiopulmonary Function Sensor]

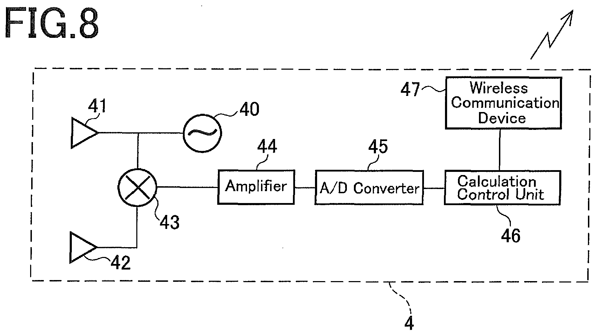

As illustrated in Fig. 8, the cardiopulmonary function sensor 4 includes a local oscillator 40, a transmitting antenna 41, a receiving antenna 42, a mixer 43, an amplifier 44, an analog-to-digital converter 45, a calculation control unit 46, and a wireless communication device 47.

The local oscillator 40 produces a microwave. The microwave produced by the local oscillator 40 is radiated from the transmitting antenna 41, is reflected by a human body (an object), and is received by the receiving antenna 42. The human body includes not only the whole body but also internal organs, such as the heart and the lungs. The microwave produced by the local oscillator 40 preferably has a frequency of about 10 MHz. This makes it easy for the microwave radiated from the transmitting antenna 41 to enter the human body and to acquire a reflected wave from the internal organs, such the heart and the lungs.

The reflected wave received by the receiving antenna 42 is mixed with a signal produced by the local oscillator 40 by the mixer 43. In other words, the microwave (a local signal) produced by the local oscillator 40 and its reflected wave (a received signal) are mixed for homodyne detection in which a Doppler signal is detected. The local oscillator 40, the transmitting antenna 41, the receiving antenna 42, and the mixer 43 are Doppler sensors, of which the mixer 43 functions as a detector.

If the human body is not moving, the microwave produced by the local oscillator 40 and the microwave reflected from the human body have the same frequency, and therefore the output of the mixer 43 contains no alternating-current component. In other words, the output of the mixer 43 is 0 Hz (a direct current).

In contrast, if the human body comes close to or away from the transmitting antenna 41 and the receiving antenna 42, the components of the reflected wave change because of the Doppler effect, and therefore a signal of the difference appears in the output of the mixer 43. The signal of the difference contains components corresponding to the motion of the heart (heartbeat) and the motion of the lungs (breathing). The output of the mixer 43 is amplified by the amplifier 44 and is converted from analog to digital by the analog-to-digital converter 45.

The calculation control unit 46 performs a fast Fourier transform on the output signal from the analog-to-digital converter 45 and calculates the heart rate and the breathing rate from the converted signal. Since the heart and the lungs exhibit different behaviors, they can be separately handled by performing a filtering process.

For example, the calculation control unit 46 performs a filtering process on the fast Fourier transformed signal using a filter in which a frequency band for a heart rate is used as a passband to calculate a heart rate from a frequency in which the amplitude is the maximum in the frequency distribution after the filtering process.

In another example, the calculation control unit 46 performs a filtering process on the fast Fourier transformed signal using a filter in which a frequency band for a breathing rate is used as a passband to calculate a breathing rate from a frequency in which the amplitude is the maximum in the frequency distribution after the filtering process.

The calculation control unit 46 converts the heart rate and the breathing rate individually into radio signals and transmits the signals to the control unit 9 of the external unit 5 via the wireless communication device 47. The control unit 9 of the external unit 5 stores the heart rate and the breathing rate in the database 7.

The database 7 stores heart rates and breathing rates so that their past histories can be ascertained as appropriate. The control unit compares the latest heart rate with the past heart rate and breathing rate, and if the difference value of the comparison result is equal to or greater than a predetermined value, the control unit instructs the display device 8 to display the occurrence of abnormality.

[Method for Correcting Blood Pressure]

In practice, upon receiving measurement data transmitted from the first blood-flow measurement sensor 2, the second blood-flow measurement sensor 3, and the cardiopulmonary function sensor 4 via the wireless communication device 6, the control unit 9 of the external unit 5 automatically stores the measurement data in the database 7.

The database 7 stores displacement data on the internal wall of a blood vessel (contraction of the inside diameter of the blood vessel) corresponding to the displacement measurement results of the blood vessel and tissue around the blood vessel due to a blood flow, heart rates and breathing rates from the cardiopulmonary function sensor 4, and pulse-wave propagation velocities and the degrees of arteriosclerosis of blood vessels (popliteal artery and thoracic aorta) acquired on the basis of all measurement data from the sensor units 12 of the first blood-flow measurement sensor 2 and the second blood-flow measurement sensor 3. The vascular properties include the percentage of the elasticity of a blood vessel, the amount of plaque in the blood vessel (a swell of intima), and the percentage of arteriosclerosis.

The blood pressure (arterial blood pressure) is expressed as: cardiac output (heart rate $\times$ one output) $\times$ blood vessel resistance (elasticity). The arterial blood pressure has the characteristic of often forming a rolling waveform during breathing particularly during dehydration in a blood vessel. Since intravascular dehydration (a decrease in the amount of circulating blood) will significantly change one output because of a change in pleural pressure due to breathing, the arterial blood pressure waveform fluctuates significantly.

Intravascular dehydration in the popliteal artery and the thoracic aorta of the measurement subject can make it difficult for the control unit 9 to accurately calculate the values of blood pressures of these arteries.

For that reason, the control unit 9 is configured to correct the calculated values of the blood pressures of the arteries by eliminating respiratory fluctuations (fluctuations in heart rate due to breathing) superposed on the arterial blood pressure waveforms of the popliteal artery and the thoracic aorta of the measurement subject as disturbance noise by using the heart rate and the breathing rate of the measurement subject.

Specifically, the control unit 9 manages all measurement data acquired from the measurement subject over a long period of time and monitors time differences in the arterial blood pressure waveform of the popliteal artery and in the arterial blood pressure waveform of the thoracic aorta. If there are variations greater than or equal to a predetermined value between the time differences between the individual arterial blood pressure waveforms, the control unit 9 corrects the time differences to substantially constant values using the heart rate and the breathing rate.

Thus, the control unit 9 can maintain the values of the blood pressures of measurement regions (popliteal artery and thoracic aorta) of the measurement subject in a state in which disturbance noise is eliminated) and thus can calculate the blood pressures with high accuracy over a long period of time.

The control unit 9 stores the values of the blood pressures of the measurement regions (popliteal artery and thoracic aorta) of the measurement subject in the database 7. The longer the management time (storage time), the more fluctuations in blood pressure that fluctuates considerably in a short time can be ascertained, so that the blood pressures can be corrected with high accuracy.

As described above, the biological-information detecting apparatus 1 is configured such that all of the first blood-flow measurement sensor 2, the second blood-flow measurement sensor 3, and the cardiopulmonary function sensor 4 are disposed in the vehicle seat S in non-contact manner. This facilitates the measurement operation without restraining the measurement subject and eliminates the attaching and detaching work unlike a method of contact with a measurement subject, allowing efficient measurement of the blood pressure in a short time.

This allows the measurement subject, who is worsening in health condition over a long period of time, to be monitored (the body condition to be ascertained) while the display device 8 of the external unit 5 is being visually monitored, dramatically increasing the probability of preventing sleeping or being frightened during driving of a vehicle.

With the biological-information detecting apparatus 1 of this embodiment, the blood pressure of the measurement subject can be measured with high accuracy only by letting the measurement subject be seated in the vehicle seat S. Since the vehicle driver is not subjected to the pressure of a cuff or restraint on the posture, burdens on the vehicle driver can be reduced.

While the above embodiment has been described with reference to a case in which the biological-information detecting apparatus 1 is used in the vehicle seat S, it is to be understood that the present invention is not limited to the above. In addition to the vehicle seat, the present invention can be broadly used in various seats with a backrest in which the first blood-flow measurement sensor 3 is disposed on the seat surface, and the second blood-flow measurement sensor 3 and the cardiopulmonary function sensor 4 are disposed on the back surface. This is effective in long-period health care of, for example, persons who are engaged in operations that need long-time seating (writers of novels and so on, the creators of games and designs, salesclerks of commodities and so on, and superintendents of apartments).

In the above embodiment, a water pack (not shown) containing water or a physiological salt solution may be provided inside the back surface of the vehicle seat S so that the second blood-flow measurement sensor 3 and the cardiopulmonary function sensor 4 are moderately pushed against the back of the measurement subject. The sensors 3 and 4 are disposed between the back of the measurement subject and the water pack. This allows the sensors 3 and 4 to be moderately pushed against the back of the measurement subject because of the elasticity of the water pack, enhancing the measurement accuracy of the blood pressure.

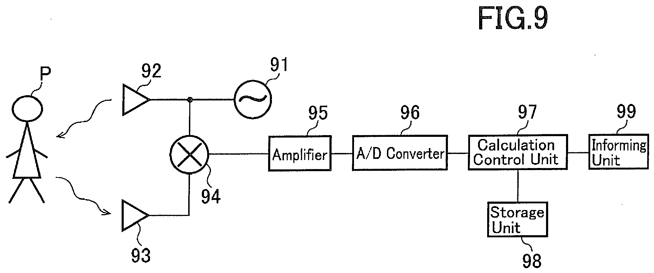

Fig. 9 illustrates, in outline, the configuration of a cardiopulmonary-function monitoring apparatus according to a second embodiment. The cardiopulmonary-function monitoring apparatus includes a local oscillator 91, a transmitting antenna 92, a receiving antenna 93, a mixer 94, an amplifier 95, an analog-to-digital converter 96, a calculation control unit 97, a storage unit 98, and an informing unit 99.

The local oscillator 91 produces a microwave. The microwave produced by the local oscillator 91 is radiated from the transmitting antenna 92, is reflected by a human body (an object) P, and is received by the receiving antenna 93. The human body P includes not only the whole body but also internal organs, such as the heart and the lungs. The microwave produced by the local oscillator 91 preferably has a frequency of about 10 MHz. This makes it easy for the microwave radiated from the transmitting antenna 92 to enter the human body P and to acquire a reflected wave from the internal organs, such the heart and the lungs.

The reflected wave received by the receiving antenna 93 is mixed with a signal produced by the local oscillator 91 by the mixer 94. In other words, the microwave (a local signal) produced by the local oscillator 91 and its reflected wave (a received signal) are mixed for homodyne detection in which a Doppler signal is detected. The local oscillator 91, the transmitting antenna 92, the receiving antenna 93, and the mixer 94 are Doppler sensors, of which the mixer 94 functions as a detector.

If the human body P is not moving, the microwave produced by the local oscillator 91 and the microwave reflected from the human body P have the same frequency, and therefore the output of the mixer 94 contains no alternating-current component. In other words, the output of the mixer 94 is 0 Hz (a direct current).

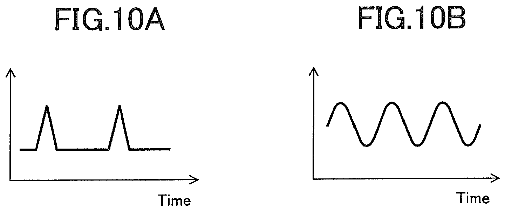

In contrast, if the human body P comes close to or away from the transmitting antenna 92 and the receiving antenna 93, the components of the reflected wave change because of the Doppler effect, and therefore a signal of the difference appears in the output of the mixer 94. The signal of the difference contains components corresponding to the motion of the heart (heartbeat) and the motion of the lungs (breathing). For example, the motion of the heart forms a signal waveform as shown in Fig. 10A, and the motion of the lungs forms a signal waveform as in Fig. 10B. Thus, the output signal from the mixer 94 contains them.

The output of the mixer 94 is amplified by the amplifier 95 and is converted from analog to digital by the analog-to-digital converter 96.

The calculation control unit 97 performs a fast Fourier transform on the output signal from the analog-to-digital converter 96 and a filtering process on the converted signal to calculate the heart rate and the breathing rate. Since the heart and the lungs exhibit different behaviors, as shown in Figs. 10A and 10B, they can be separately handled by performing a filtering process.

For example, the calculation control unit 97 performs a filtering process on the fast Fourier transformed signal using a filter in which a frequency band for a heart rate is used as a passband to calculate a heart rate from a frequency in which the amplitude is the maximum in the frequency distribution after the filtering process.

In another example, the calculation control unit 97 performs a filtering process on the fast Fourier transformed signal using a filter in which a frequency band for a breathing rate is used as a passband to calculate a breathing rate from a frequency in which the amplitude is the maximum in the frequency distribution after the filtering process.

The storage unit 98 stores the heart rate and the breathing rate calculated by the calculation control unit 97.

The calculation control unit 97 compares the average value of past heart rates and breathing rates stored in the storage unit 98 with the calculated latest heart rate and breathing rate, and if the difference is equal to or greater than a predetermined value, the calculation control unit 97 instructs the informing unit 99 to give an alarm about the occurrence of abnormality. Alternatively, the calculation control unit 97 may compare reference values of heart rates and breathing rates stored in the storage unit 98 with the calculated latest heart rate and breathing rate, and if the difference is equal to or greater than a predetermined value, the calculation control unit 97 may instruct the informing unit 99 to give an alarm about the occurrence of abnormality.

The informing unit 99 gives an alarm about the occurrence of abnormality in the heartbeat or breathing according to an instruction from the calculation control unit 97. For example, the informing unit 99 generates alarm sound or causes a monitor display to display the occurrence of abnormality.

Thus, this embodiment allows a cardiopulmonary function to be monitored without a sensor or the like brought into direct-contact with the human body. Furthermore, this embodiment allows the occurrence of abnormality in the cardiopulmonary function to be quickly detected and informed as it is.

(Example 1)

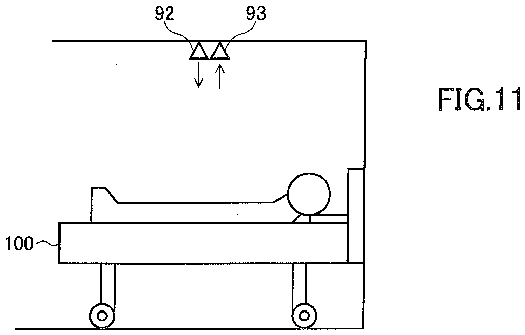

The transmitting antenna 92 and the receiving antenna 93 of the cardiopulmonary-function monitoring apparatus according to the second embodiment can be disposed several meters (for example, about one to two meters) away from a human body. This allows, for example, as illustrated in Fig. 11, the transmitting antenna 92 and the receiving antenna 93 to be disposed on a ceiling above a bed 100 on which a monitored object is sleeping or a wall surface beside the bed 100, thereby allowing the cardiopulmonary function of the person who is sleeping on the bed 100 to be monitored.

(Example 2)

The transmitting antenna 92 and the receiving antenna 93 of the cardiopulmonary-function monitoring apparatus according to the second embodiment can be disposed several meters away from the human body, as described above. For that reason, the transmitting antenna 92 and the receiving antenna 93 may be disposed, for example, on the ceiling or a wall surface of a room in which a monitored object spends a certain period of time in a seated position or a standing position. This allows the cardiopulmonary function of a person who is waiting in a room, such as a waiting room of a hospital, to be monitored. This allows, for example, information regarding the cardiopulmonary function of a patient, to be acquired before diagnosis, thereby enhancing the efficient of the diagnosis.

(Example 3)

The transmitting antenna 92 and the receiving antenna 93 of the cardiopulmonary-function monitoring apparatus according to the second embodiment can be disposed several meters away from a human body, as described above. For that reason, the transmitting antenna 92 and the receiving antenna 93 may be disposed, for example, on the ceiling or a wall surface of a house or a workplace of a monitored object. This allows the cardiopulmonary function of a monitored object in daily life to be monitored. A plurality of pairs of transmitting antenna 92 and receiving antenna 93 may be disposed in each room.

(Example 4)

The transmitting antenna 92 and the receiving antenna 93 of the cardiopulmonary-function monitoring apparatus according to the second embodiment can be disposed several meters away from a human body, as described above. For that reason, the transmitting antenna 92 and the receiving antenna 93 may be disposed on the ceiling or a wall surface of a public facility (for example, classrooms of schools, libraries, and gymnasiums). This allows the cardiopulmonary-function monitoring apparatus to be utilized as an infrastructure for monitoring the cardiopulmonary functions of the users of the facility.

Disposing the transmitting antenna 92 and the receiving antenna 93 of the cardiopulmonary-function monitoring apparatus according to the second embodiment on the ceiling or a wall surface of a space in which a monitored object stays in a recumbent, seated, or standing position, such as a bedroom of his/her house, a hospital room or a waiting room, an office of a workplace, and a public facility, allows the cardiopulmonary function of a monitored object, such as an inhabitant, a patient, an employee, and a facility user, to be monitored.

The configuration of the cardiopulmonary-function monitoring apparatus according to the second embodiment is not limited to the configuration in which the transmitting antenna 92 and the receiving antenna 93 are fixedly disposed on a ceiling or a wall surface. The transmitting antenna 92 and the receiving antenna 93 may be equipped with an engaging portion having a shape engageable with a fixed surface, such as a ceiling or a wall, or a fixing means using a magnet so as to be attached to or detached from the ceiling or the wall. This allows the cardiopulmonary-function monitoring apparatus according to the present invention to be carried separately.

At least the transmitting antenna 92 and the receiving antenna 93 of the cardiopulmonary-function monitoring apparatus according to the second embodiment may be built in another installation instrument. Another installation instrument refers to an instrument for use other than the monitoring of the cardiopulmonary function, for example, lighting fixtures, such as a ceiling light, home electric appliances, such as audio instruments and TV sets, furniture, and appointments.

The other components of the cardiopulmonary-function monitoring apparatus (at least any of the mixer 94, the amplifier 95, the analog-to-digital converter 96, the calculation control unit 97, the storage unit 98, and the informing unit 99) may be built in the installation instrument.

(Embodiment 5)

At least the transmitting antenna 92 and the receiving antenna 93 of the cardiopulmonary-function monitoring apparatus according to the second embodiment may be built in an apparatus that the user (a monitored object) can carry (a portable device). This portable device has such a size and shape that the user (a monitored object) can carry. Examples include mobile electronic devices, such as smartphones and tablet terminals. This allows the cardiopulmonary function to be monitored at a remote location.

A server that is communicably connected to the portable device may be provided. The calculation control unit 97 may be disposed in the server. In this case, an output signal from the analog-to-digital converter 96 is transmitted to the server, and the calculation control unit 97 of the server calculates the heart rate and the breathing rate and transmits the rates to the portable device.

Having described the present invention in detail using specific configurations, it is obvious to those skilled in the art that various modifications can be made without departing from the intention and scope of the present invention. The present application is based on Japanese Patent Application No. 2014-136082 filed July 1, 2014 and No. 2015-049591 filed March 12, 2015, which are hereby incorporated by reference herein in their entirety.

Reference Signs List

| | |
|---|---|
| [0138] | 1 BIOLOGICAL-INFORMATION DETECTING APPARATUS |
| 2 | FIRST BLOOD-FLOW MEASUREMENT SENSOR |
| 3 | SECOND BLOOD-FLOW MEASUREMENT SENSOR |
| 4 | CARDIOPULMONARY FUNCTION SENSOR |
| 5 | EXTERNAL UNIT |
| 6, 14, 47 | WIRELESS COMMUNICATION DEVICE |
| 7 | DATABASE |
| 8 | DISPLAY DEVICE |
| 9 | CONTROL UNIT |
| 12 | SENSOR UNIT |
| 13 | MEASUREMENT CONTROL UNIT |
| 91 | LOCAL OSCILLATOR |
| 92 | TRANSMITTING ANTENNA |
| 93 | RECEIVING ANTENNA |
| 94 | MIXER |
| 95 | AMPLIFIER |
| 96 | ANALOG-TO-DIGITAL CONVERTER |
| 97 | CALCULATION CONTROL UNIT |
| 98 | STORAGE UNIT |
| 99 | INFORMING UNIT |

**Claims**

1. A biological-information detecting apparatus comprising:

first blood-flow measuring means provided at a seat surface of a seat, the first blood-flow measuring means measuring a condition of a blood flow of a popliteal artery of a measurement subject seated in the seat;

second blood-flow measuring means provided at a back of the seat, the second blood-flow measuring means measuring a condition of a blood flow of a thoracic aorta of the measurement subject; and

blood-pressure calculation means determining a pulse-wave propagation velocity and a degree of arteriosclerosis from blood flow data acquired by the first blood-flow measuring means and blood flow data acquired by the second blood-flow measuring means and calculating blood pressures of the popliteal artery and the thoracic aorta of the measurement subject based on the pulse-wave propagation velocity and the degree of arteriosclerosis.

2. The biological-information detecting apparatus according to Claim 2, further comprising:

a database storing the blood flow data acquired from the first blood-flow measuring means and the second blood-flow measuring means,

wherein the blood-pressure calculation means calculates the blood pressures of the popliteal artery and the thoracic aorta in chronological order based on the individual blood flow data stored in the database over a predetermined period of time to monitor a health condition of the measurement subject.

3. The biological-information detecting apparatus according to Claim 1 or 2, further comprising:

cardiopulmonary-function measuring means provided at the back of the seat, the cardiopulmonary-function measuring means measuring a heart rate and a breathing rate of the measurement subject,

wherein the blood-pressure calculation means individually corrects the blood pressures of the popliteal artery and the thoracic aorta based on the heart rate and the breathing rate so that time differences in the waveform of the pressure of the popliteal artery and in the waveform of the thoracic aorta of the measurement subject become substantially constant.

4. The biological-information detecting apparatus according to Claim 3,

wherein the database stores heart rates and breathing rates acquired from the cardiopulmonary-function measuring means, and

wherein the blood-pressure calculation means corrects the blood pressures of the popliteal artery and the thoracic aorta based on the heart rates and the breathing rates stored in the database over the predetermined period.

5. A seat with a backrest comprising the biological-information detecting apparatus according to any of Claims 1 to 4.

6. A cardiopulmonary-function monitoring apparatus comprising:

an oscillator producing a microwave;

a transmitting antenna transmitting the microwave produced by the oscillator;

a receiving antenna receiving a reflected wave from a monitored object;

a mixer mixing the microwave produced by the oscillator with the reflected wave to output a signal based on a difference between the microwave and the reflected wave;

an analog-to-digital converter converting the signal output from the mixer from analog to digital; and

a calculation control unit performing a Fourier transform on the signal output from the analog-to-digital converter and performing a filtering process on the Fourier transformed signal to calculate a heart rate and a breathing rate.

7. The cardiopulmonary-function monitoring apparatus according to Claim 6, wherein the calculation control unit calculates the heart rate based on a frequency whose amplitude is maximum in a frequency distribution after a filtering process using a first filter corresponding to a heartbeat and calculates the breathing rate based on a frequency whose amplitude is maximum in a frequency distribution after a filtering process using a second filter corresponding to breathing.

8. The cardiopulmonary-function monitoring apparatus according to Claim 6 or 7, further comprising:

an informing unit giving an alarm of occurrence of abnormality,

wherein, when a difference between the calculated heart rate or breathing rate and a reference value is greater than or equal to a predetermined value, the calculation control unit instructs the informing unit to given an alarm of the occurrence of abnormality.

**9.** The cardiopulmonary-function monitoring apparatus according to Claim 8, further comprising:

a storage unit storing heart rates and breathing rates calculated by the calculation control unit,
wherein the calculation control unit uses an average value of the heart rates or the breathing rates stored in the storage unit as the reference value.

**10.** The cardiopulmonary-function monitoring apparatus according to any of Claims 6 to 9, wherein the microwave produced by the oscillator has a frequency of 10 MHz.

**11.** The cardiopulmonary-function monitoring apparatus according to any of Claims 6 to 10, wherein the transmitting antenna and the receiving antenna are mounted on a ceiling or a side wall surface of a space in which the monitored object stays in a recumbent position, a seated position, or a standing position.

**12.** The cardiopulmonary-function monitoring apparatus according to any of Claims 6 to 10, wherein the transmitting antenna and the receiving antenna are configured to be attachable to or detachable from a ceiling or a wall surface of a space in which the monitored object stays in a recumbent position, a seated position, or a standing position.

**13.** The cardiopulmonary-function monitoring apparatus according to any of Claims 6 to 10, wherein at least the transmitting antenna and the receiving antenna are built in another installation instrument.

**14.** The cardiopulmonary-function monitoring apparatus according to any of Claims 6 to 10, wherein at least the transmitting antenna and the receiving antenna are built in a portable device.

FIG.1

FIG.2

Wireless Communication Device — 14

Measurement Control Unit — 13

Battery — 15

Sensor Unit — 12

16

2,3

11

10

FIG.3

FIG.4

**FIG.5**

Light Absorptance($10^{-6}$ cm$^2$,mol$^{-1}$) vs Wavelength [$\mu$m]

——— HbO2 (Hemoglobin Oxide)
- - - - Hb (Hemoglobin)

# FIG.6A

Detection Signal From X1
Photosensor 21A

Ts T1

# FIG.6B

Detection Signal From X2
Photosensor 21B

$(x_1 > x_2)$
$(T_1 < T_2)$

Ts T2

# FIG.6C

Detection Signal From X3
Photosensor 21C

$(x_2 > x_3)$
$(T_2 < T_3)$

Ts          T3

**FIG.7**

```
            ( PWV Control Process )
                      │
   ┌──────────────────┤
   │    ┌─────────────▼──────────────────┐
   │    │ Read Measurement Data from Database │──S1
   │    └─────────────┬──────────────────┘
   │                  │
   │    ┌─────────────▼──────────────────┐
   │    │ Calculate Concentration of Red Blood Cells │──S2
   │    │ from Measurement Data           │
   │    └─────────────┬──────────────────┘
   │                  │
   │    ┌─────────────▼──────────────────┐
   │    │ Derive Vascular Properties from Changes in │──S3
   │    │ Blood Flow at Individual Measuremt Positions │
   │    └─────────────┬──────────────────┘
   │                  │
   │    ┌─────────────▼──────────────────┐
   │    │ Compare Detection Signal Waveforms │──S4
   │    └─────────────┬──────────────────┘
   │                  │
   │    ┌─────────────▼──────────────────┐
   │    │ Derive PWV Properties from Phase Differences │──S5
   │    │ between Waveforms               │
   │    └─────────────┬──────────────────┘
   │                  │
   │    ┌─────────────▼──────────────────┐
   │    │ Store and Display Derived PWV Properties │──S6
   │    └─────────────┬──────────────────┘
   │                  │           S7
   │           ╱──────▼──────╲
   │    No    ╱  Has           ╲
   └─────────◄  Calculation for All ►
             ╲  Measurement Data been ╱
              ╲  Completed?  ╱
               ╲──────┬──────╱
                    Yes
   ┌──────────────────▼──────────────────┐
   │ Display PWV Measurement Results on Screen │──S8
   └──────────────────┬──────────────────┘
                      │
                 ( Return )
```

# FIG.8

FIG.9

P

92    91

95    96    97    99

| Amplifier | A/D Converter | Calculation Control Unit | Informing Unit |

94

93

Storage Unit

98

FIG.10A

FIG.10B

Time

Time

92    93

100

FIG.11

# EP 3 165 157 A1

## INTERNATIONAL SEARCH REPORT

| International application No. |
| --- |
| PCT/JP2015/068787 |

**A. CLASSIFICATION OF SUBJECT MATTER**
*A61B5/022*(2006.01)i, *A61B5/0245*(2006.01)i, *A61B5/026*(2006.01)i, *A61B5/08*
(2006.01)i, *A61B5/11*(2006.01)i

According to International Patent Classification (IPC) or to both national classification and IPC

**B. FIELDS SEARCHED**

Minimum documentation searched (classification system followed by classification symbols)
A61B5/02, A61B5/08, A61B5/11

Documentation searched other than minimum documentation to the extent that such documents are included in the fields searched
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)
JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2013-106641 A (University of Tsukuba), 06 June 2013 (06.06.2013), paragraphs [0024] to [0071]; fig. 4, 10 (Family: none) | 1-5 |
| A | JP 2014-76226 A (Panasonic Corp.), 01 May 2014 (01.05.2014), paragraphs [0036] to [0038], [0055] to [0068]; fig. 1, 4 to 5 (Family: none) | 1-5 |
| A | JP 2009-172204 A (Equos Research Co., Ltd.), 06 August 2009 (06.08.2009), paragraphs [0018], [0037]; fig. 1 to 2, 4 (Family: none) | 1-5 |

☒ Further documents are listed in the continuation of Box C.   ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 16 September 2015 (16.09.15) | 06 October 2015 (06.10.15) |

| Name and mailing address of the ISA/ | Authorized officer |
| --- | --- |
| Japan Patent Office<br>3-4-3,Kasumigaseki,Chiyoda-ku,<br>Tokyo 100-8915,Japan | |
| | Telephone No. |

Form PCT/ISA/210 (second sheet) (July 2009)

**INTERNATIONAL SEARCH REPORT**

| International application No. |
| --- |
| PCT/JP2015/068787 |

C (Continuation).  DOCUMENTS CONSIDERED TO BE RELEVANT

| Category* | Citation of document, with indication, where appropriate, of the relevant passages | Relevant to claim No. |
| --- | --- | --- |
| A | JP 2008-99849 A (Kabushiki Kaisha Tau Giken), 01 May 2008 (01.05.2008), claim 1 (Family: none) | 3-4 |

Form PCT/ISA/210 (continuation of second sheet) (July 2009)

**INTERNATIONAL SEARCH REPORT**

| International application No. |
| --- |
| PCT/JP2015/068787 |

---

**Box No. II**     **Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

---

**Box No. III**     **Observations where unity of invention is lacking (Continuation of item 3 of first sheet)**

This International Searching Authority found multiple inventions in this international application, as follows:
    See extra sheet.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.  Consequently, this international search report is restricted to the invention first mentioned in the claims; it is covered by claims Nos.:

   Claims 1-5

**Remark on Protest**     ☐ The additional search fees were accompanied by the applicant's protest and, where applicable, the payment of a protest fee.

    ☐ The additional search fees were accompanied by the applicant's protest but the applicable protest fee was not paid within the time limit specified in the invitation.

    ☐ No protest accompanied the payment of additional search fees.

Form PCT/ISA/210 (continuation of first sheet (2)) (July 2009)

| **INTERNATIONAL SEARCH REPORT** | International application No. |
| --- | --- |
| | PCT/JP2015/068787 |

Continuation of Box No.III of continuation of first sheet(2)

Claims 1 and 6 have a common technical feature of a bioinformation detection device. However, this technical feature does not contribute to prior art on comparison with the details disclosed in Document 1 (JP 2013-106641 A (University of Tsukuba), 06 June 2013 (06.06.2013), paragraphs [0024] to [0071]; fig. 4, 10), Document 2 (JP 2014-76226 A (Panasonic Corp.), 01 May 2014 (01.05.2014), paragraphs [0036] to [0038], [0055] to [0068]; fig. 1, 4 to 5), Document 3 (JP 2009-172204 A (Equos Research Co., Ltd.), 06 August 2009 (06.08.2009), paragraphs [0018], [0037]; fig. 1 to 2, 4), and Document 4 (JP 2008-99849 A (Kabushiki Kaisha Tau Giken), 01 May 2008 (01.05.2008), claim 1). Therefore this technical feature is not a special technical feature. Moreover, there are no other same or corresponding special technical features between these inventions. The claims are categorized in two inventions having the following respective special technical features.

Invention 1: Claims 1-5
A bioinformation detection device including: first blood flow measurement means which is provided on a seat part of a seat and measures a blood flow state of the popliteal artery of a subject seated on the seat; second blood flow measurement means which is provided on a back part of the seat and measures a blood flow state of the thoracic aorta of the subject; and blood pressure calculation means which obtains pulse wave velocity and a degree of arteriosclerosis from blood flow data measured by the first blood flow measurement means and blood flow data measured by the second blood flow measurement means, and calculates blood pressure of the popliteal artery and the thoracic aorta of the subject on the basis of the pulse wave velocity and the degree of arteriosclerosis.

Invention 2: Claims 6-14
A cardiopulmonary monitor including: an oscillator that generates microwaves; a transmission antenna that transmits the microwaves generated by the oscillator; a reception antenna that receives reflected waves from a subject being monitored; a mixer that mixes the micro waves generated by the oscillator and the reflected waves and outputs a signal based on a difference between the microwaves and the reflected waves; an A/D converter that A/D converts the signal output from the mixer; and a calculation control unit that Fourier transforms the signal output from the A/D converter, subjects the Fourier transformed signal to a filtering process, and calculates heart rate and respiration rate.
Claims 6-14 are not inventions in the same category that includes all the matters defining the invention of Claim 1. As a result of a search on the claims categorized as Invention 1, it was found that Claims 6-14 are not inventions for which a search is possible without substantially additional prior art search or judgments and there are no other reasons to consider it efficient to conduct a search for Claims 6-14 together with Claims 1-5. Therefore, Claims 6-14 cannot be categorized as Invention 1.

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007290504 A
- WO 200746283 A
- JP 2008099849 A
- JP 2014000105 A
- JP 2004174168 A
- JP 2014136082 A
- JP 2015049591 A